(19) Europäisches Patentamt
European Patent Office
Office européen des brevets

(11) Veröffentlichungsnummer : **0 146 007**
**B1**

(12) # EUROPÄISCHE PATENTSCHRIFT

(45) Veröffentlichungstag der Patentschrift : **30.12.86**

(51) Int. Cl.⁴ : **C 07 C147/12**, C 07 C147/10, C 07 C149/36

(21) Anmeldenummer : **84114173.2**

(22) Anmeldetag : **23.11.84**

(54) **Verfahren zur Herstellung von 5-Oxethylsulfonyl-2-amino-phenol(-ethern).**

(30) Priorität : **01.12.83 DE 3343421**

(43) Veröffentlichungstag der Anmeldung : **26.06.85 Patentblatt 85/26**

(45) Bekanntmachung des Hinweises auf die Patenterteilung : **30.12.86 Patentblatt 86/52**

(84) Benannte Vertragsstaaten : **CH DE FR GB IT LI**

(56) Entgegenhaltungen :
**EP-A- 0 092 909**
**FR-A- 2 360 539**
**GB-A- 1 195 344**

(73) Patentinhaber : **HOECHST AKTIENGESELLSCHAFT**
**Postfach 80 03 20**
**D-6230 Frankfurt am Main 80 (DE)**

(72) Erfinder : **Papenfuhs, Theodor, Dr.**
**Heinrich-Bleicher-Strasse 40**
**D-6000 Frankfurt am Main 50 (DE)**

**EP 0 146 007 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der vorliegenden Erfindung ist ein technisch vorteilhaftes, nur wenige Stufen umfassendes, im Vergleich zum bekannten Verfahren die Umwelt geringer belastendes Verfahren zur Herstellung von 5-Oxethylsulfonyl-2-aminophenol(-ethern) der allgemeinen Formel (I)

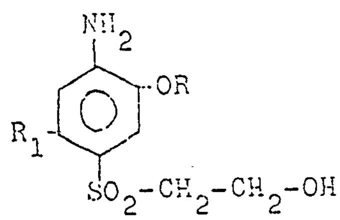

in welcher R ein Wasserstoffatom oder eine Alkylgruppe von 1-4 Kohlenstoffatomen, und $R_1$ ein Wasserstoffatom oder eine Alkyl- oder Alkoxygruppe von jeweils 1-4 Kohlenstoffatomen bedeuten.

5-Oxethylsulfonyl-2-aminophenol(-ether) der vorstehend genannten allgemeinen Formel (I) sind wichtige Farbstoff-Vorprodukte und dienen insbesondere zur Herstellung von Reaktivfarbstoffen. Ihre technische Herstellung erfolgt bisher im Falle des 5-Oxethylsulfonyl-2-aminophenols ($R = R_1 = H$) aus o-Aminophenol, das mit Harnstoff zu Benzoxazolen cyclisiert (Schutz von Amino- und Hydroxyfunktion), mit überschüssiger Chlorsulfonsäure (ggf. in Gegenwart von Thionylchlorid) sulfochloriert, anschließend mit Bisulfit/Natronlauge reduziert und mit Ethylenoxid kondensiert, sowie letztlich unter Ringhydrolyse mittels Schwefelsäure zum 5-Oxethylsulfonyl-2-aminophenol (bzw. seinem Sulfatester) umgesetzt wird gemäß nachstehendem Reaktionsschema :

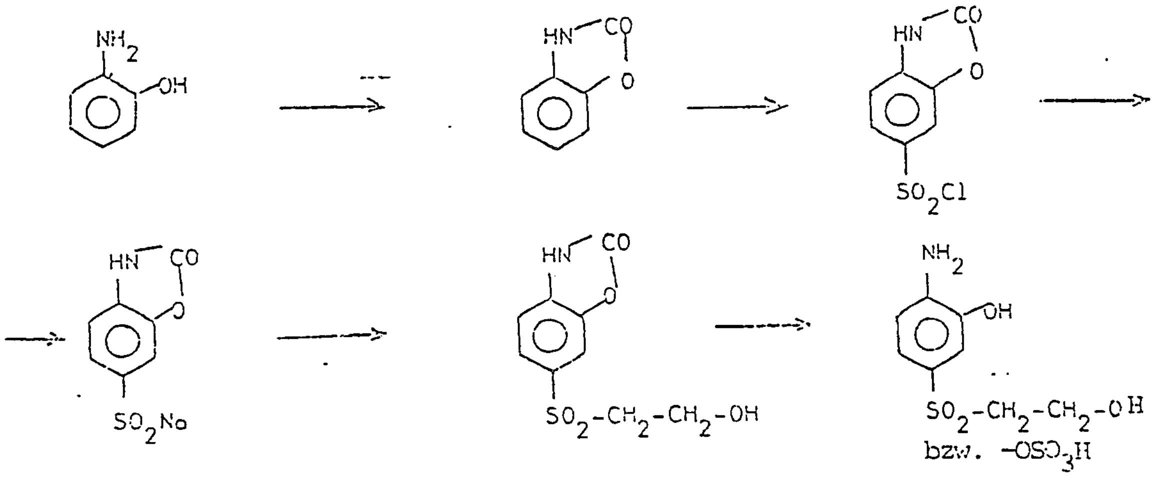

Im Falle der entsprechenden Phenolether (R = Alkyl in Formel I) erfolgt die technische Herstellung bisher durch Acylierung des zugrundeliegenden Amins, der sich die oben beschriebenen Folgereaktionen in analoger Weise anschließen, so daß man letztlich zu den 5-Oxethylsulfonyl-2-aminophenolethern (bzw. ihren Sulfatestern) gelangt gemäß folgendem Reaktionsschema :

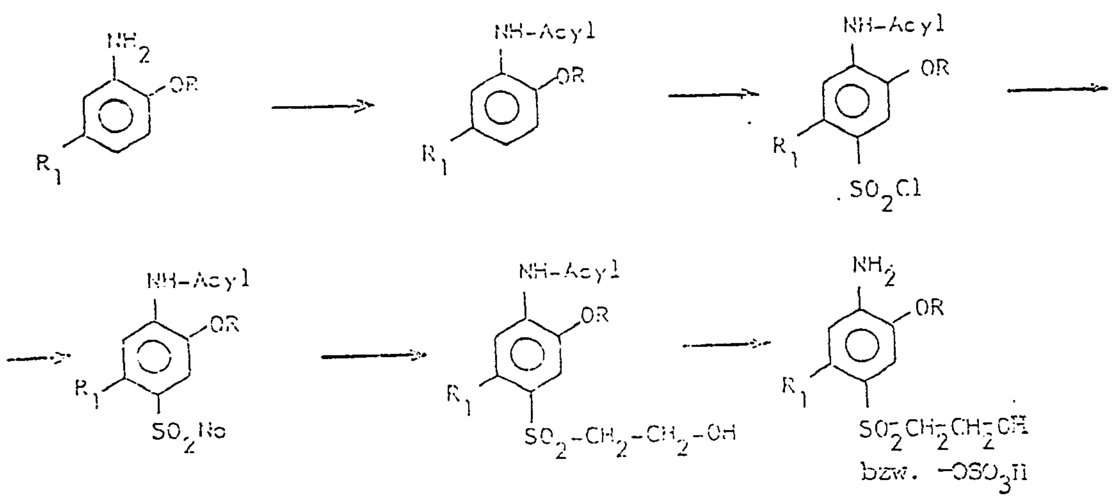

Diese vielstufigen Verfahren sind technisch sehr aufwendig, benötigen meist spezielle Apparaturen und zeichnen sich durch eine hohe Belastung des Abwassers (Salz- und Schwefelsäure aus der Sulfochlorierung, Salze aus der Sulfitreduktion, Ethylenoxid-Folgeprodukte) aus. Sie sind daher nicht wirtschaftlich.

Es bestand infolgedessen die Aufgabe, diese wichtigen Farbstoff-Vorprodukte auf ökonomischerem Wege in technisch vorteilhafter, nur wenige Stufen umfassender Synthese und unter Verringerung der Umweltbelastung zugänglich zu machen.

Diese Aufgabe wird durch vorliegende Erfindung gelöst, indem man die technisch durch partielle Hydrolyse bzw. Alkoholyse von 2,4-Dichlor- bzw. -Dibromnitrobenzolen leicht zugänglichen 5-Chlor (bzw. Brom)-2-nitrophenole bzw. deren Alkylether, sowie die durch Nitrierung von Halogen-hydrochinondialkylethern technisch verfügbaren 5-Chlor-(bzw. Brom)-2-nitrohydrochinon-dialkylether in hohen Ausbeuten, mit Thioglykol zu 2-Nitrophenol(ether)-5-oxyethylsulfiden in rein wäßrigem Medium kondensiert und diese mit oder ohne, vorzugsweise ohne Zwischenisolierung, zu 5-Oxethylsulfonyl-2-nitrophenol-(ethern) oxidiert. Abschließende Reduktion der Nitrogruppe liefert die gesuchten Zielprodukte der Formel (I) in einer minimalen Anzahl von Synthesestufen sowie in hoher Ausbeute und Qualität.

Die vorliegende Erfindung betrifft somit ein Verfahren zur Herstellung von 5-Oxethylsulfonyl-2-aminophenol(-ethern) der allgemeinen Formel (I)

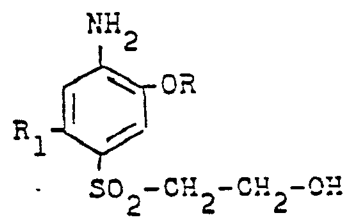

(I)

in welcher R ein Wasserstoffatom oder eine Alkylgruppe von 1-4 Kohlenstoffatomen, und $R_1$ ein Wasserstoffatom oder eine Alkyl- oder Alkoxygruppe von jeweils 1-4 Kohlenstoffatomen bedeuten, dadurch gekennzeichnet, daß man 5-Chlor-(oder Brom)-2-nitrophenole oder deren Mono-Alkylether der allgemeinen Formel (II)

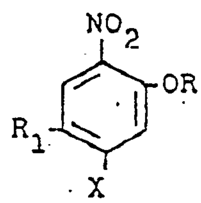

(II)

in welcher R und $R_1$ die vorstehend genannten Bedeutungen haben, X ein Chlor- oder Bromaton bedeutet, mit Thioglykol zu 2-Nitrophenol(-ether)-5-oxethylsulfiden der Formel (III)

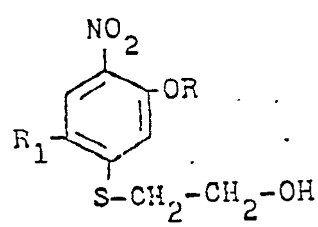

(III)

in welcher R und $R_1$ die vorstehend genannten Bedeutungen haben, in rein wäßrigem Medium kondensiert, diese mit oder vorzugsweise ohne Zwischenisolierung zu 5-Oxethylsulfonyl-2-nitrophenol (-ethern) der Formel (IV)

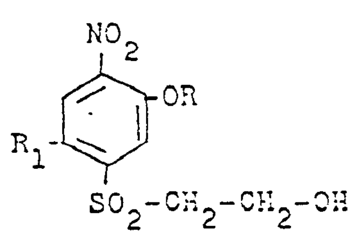

(IV)

oxidiert und letztere zu den Verbindungen der genannten Formel (I) reduziert.

Die Umsetzung von durch Nitrogruppen aktivierten Halogenatomen mit Thioglykol zu Nitroaryloxethylsulfiden ist zwar im Prinzip bekannt (vgl. DE-OS 3 135 367) und gelingt bei o-Nitrohalogenbenzolen in hohen Ausbeuten. Bei den p-Isomeren wird dagegen eine unübersichtliche Reaktion beschrieben (vgl. J. Chem. Soc. *1927*, 1668), die bei optimaler Durchführung (gemäß DE-OS 3 135 367) allenfalls mit mäßigen Ausbeuten (60-75 %) das gesuchte 4-Nitrooxethylsulfid liefert, sofern nicht technisch ungünstige, da technisch aufwendig zu regenerierende dipolar aprotische Lösungsmittel (Dimethylformamid oder -acetamid, N-Methylpyrrolidon bzw. -imidazolidinon) zum Einsatz kommen.

Es war daher überraschend und nicht vorauszusehen, daß p-Nitro-halogenbenzole, die durch halogen-desaktivierende Gruppen (Alkyl-, Alkoxy-, Hydroxygruppen) noch ungünstiger substituiert sind (vgl. Formel II), selbst in Abwesenheit jeglicher Lösungsmittel, d. h. in wäßriger Lösung oder Suspension, mit Thioglykol in hohen Ausbeuten und Reinheiten zu den gesuchten Thioethern der Formel (III) kondensiert werden können.

Auch die Oxidation von Aryl-alkyl-thioethern zu den korrespondierenden Sulfonen ist im Prinzip bekannt (vgl. DBP 944 607, Oxidationsmittel Hypochlorit in alkalischer Lösung ; JP-AS 24 661/68, Oxidationsmittel Wasserstoffperoxid in Gegenwart von Bicarbonaten ; DE-OS 3 135 367, Oxidationsmittel Wasserstoffperoxid in Gegenwart katalytischer Mengen Wolframsäure). Die Anwendung der aus DBP 944 607 und JP-AS 24 661/68 bekannten Oxidationsverfahren, die in alkalischem Milieu ablaufen, führt bei Einsatz der erfindungsgemäß ausgewählten Thioether der Formel (III) überhaupt nicht zum Ziel. Es werden ausnahmslos in unübersichtlicher Reaktion Zersetzungs- und/oder Oxidationsprodukte der Ausgangsverbindungen erhalten. Die gesuchten 5-Oxethylsulfonyl-2-nitrophenol(-ether) der Formel (IV) werden in keinem Falle in isolierbaren Mengen gebildet. Ein ähnlich entmutigendes Ergebnis wird erhalten, wenn das aus der DE-OS 3 135 367 bekannte Verfahren bei pH-Werten $\geq 6$ (beansprucht wird ein pH-Bereich von 3-8) durchgeführt wird.

Dagegen erhält man eine glatte, in hoher Ausbeute ablaufende Oxidation der Thioether der Formel (III) zu den Oxethylsulfonen der Formel (IV), wenn die Umsetzung mit Wasserstoffperoxid in Gegenwart von katalytischen Mengen Wolframsäure in einem pH-Bereich von 4-6 durchgeführt wird. Diese drastische Änderung des Oxidationsverlaufs bei nur geringfügiger pH-Änderung ist an sich schon überraschend. Noch überraschender ist aber, daß Phenole und Phenolether, wie sie die Verbindungen der Formel (III) ausnahmslos darstellen, unter bestimmten Reaktionsbedingungen ohne Kernhydroxylierung mit $H_2O_2$ umgesetzt werden können. Es konnte nämlich erwartet werden, daß in literaturbekannter Weise (vgl. Houben-Weyl 6, 1c, S. 30 ff) bei Einwirkung von Wasserstoffperoxid auf Hydroxy- oder Alkoxyphenyloxethyl-sulfide (Formel III) neben oder an Stelle der Oxidation der Schwefelbrücke eine Hydroxylierung des Phenylkerns erfolgt, wodurch unübersichtliche Produktgemische und damit unbefriedigende Ausbeuten resultieren würden. Außerdem war von vornherein auch eine Hydrolyse der zur Nitrogruppe o-ständigen Alkoxygruppe in den Thioethern der Formel (III) (R = Alkyl) bei Umsetzung in wasserhaltigen Systemen nicht auszuschließen.

Solche Nebenreaktionen laufen vermutlich, wahrscheinlich pH-abhängig, auch bei der Umsetzung der Thioether der Formel (III) mit Wasserstoffperoxid neben- und/oder nacheinander ab und führen in pH-Bereichen $< 4$ und $> 6$ unter anderem zu einem völlig unübersichtlichen Reaktionsgeschehen, welches ein Erreichen des Ziels, die einheitliche Isolierung der gesuchten Endprodukte der Formel (IV) zu optimieren, wenig wahrscheinlich erscheinen ließ.

Es war daher umso überraschender, daß es gelingt, im pH-Bereich von 4-6 in wäßriger lösung oder Suspension die angestrebte Oxidation der Thioetherbrücke in 2-Nitrophenol-(ether)-5-oxethylsulfiden (Formel III) selektiv und vollständig durchzuführen und bei der wolframat-katalysierten Umsetzung dieser Verbindungen der Formel (III) mit mindest stöchiometrischer Menge (2 Mol) Wasserstoffperoxid die gesuchten 5-Oxethylsulfonyl-2-nitro-phenol(-ether) (Formel IV) in hoher Ausbeute zu erhalten.

Der erfindungsgemäß pH-Bereich schließt bei Einsatz hydroxylgruppenhaltiger Ausgangsprodukte (Formel II, R = H) aus, daß die desaktivierende Wirkung der OH-Gruppe durch Salzbildung aufgehoben wird, so daß der glatte Ablauf der Oxidation erst recht nicht erwartet werden konnte. Das erfindungsgemäße Verfahren ist daher für die beanspruchten Zielprodukte neu, überraschend und dem Stand der Technik ökonomisch und ökologisch überlegen und stellt somit einen erheblichen technischen Fortschritt dar.

Das Verfahren wird im einzelnen in der Weise durchgeführt, daß man ein gemäß Formel (II) substituiertes p-Nitro-halogenbenzol zusammen mit einer mindestens stöchiometrischen Menge Thioglykol und mit Wasser (zur Erzielung einer ausreichenden Rührbarkeit in jeder Phase der Umsetzung) auf Temperaturen von 50-90 °C, vorzugsweise 65-80 °C erhitzt und dann im Verlaufe von 1-5 Stunden, vorzugsweise 2-3 Stunden, anteilweise eine mindestens stöchiometrische Menge einer säurebindenden Alkali- oder Erdalkalimetallverbindung, wie beispielsweise ein Alkali- oder Erdalkalimetalloxid, -hydroxid oder -carbonat, vorzugsweise Natrium- oder insbesondere Kaliumcarbonat (bei Einsatz von Verbindungen der Formel (II) mit R = H 1 Mol Carbonat pro Mol Verbindung der Formel (II), bei Einsatz von Verbindungen der Formel (II) mit $R \neq H$ 0,5 Mol Carbonat pro Mol Verbindung der Formel (II)) einträgt. Anschließend wird bis zur Beendigung der Umsetzung (Kontrolle durch Dünnschichtchromatografie oder HPLC) (HPLC = High performance liquid chromatography analysis) nachgerührt (3-10 Stunden) und

dann durch Zulauf von Säure (Mineral- oder Essigsäure) ein pH-Wert von 4-6 eingestellt. Das gebildete 2-Nitrophenol(-ether)-5-oxethylsulfid (Formel III) kann nach Abkühlen auf Raumtemperatur durch Filtration in hoher Ausbeute isoliert werden. Vorteilhafter ist es jedoch, die erhaltene Suspension nach Zusatz katalytischer Mengen Wolframtrioxid oder Alkalimetallwolframat (1-10 g pro Mol Ausgangsverbindung der Formel (II)) und ggf. nach Verdünnung mit Wasser mit der mindest stöchiometrischen Menge Wasserstoffperoxid (2 Mol pro Mol Verbindung der Formel III) zu versetzen und nach beendeter Oxidation (Kontrolle durch DC (= Dünnschichtchromatografie) oder HPLC, Nachrührzeit ca. 2-6 Stunden) aus der erhaltenen Lösung oder Suspension das Produkt der Formel IV durch Kühlen auf 0-20 °C auszukristallisieren und nachfolgend durch Filtration zu isolieren.

Die abschließende Reduktion der Nitrogruppe ist unkritisch. Sie kann nach bekannten Methoden, beispielsweise durch Eisen- oder katalytische Reduktion an Nickel- oder Edelmetall-Katalysatoren in wäßrigem System durchgeführt werden und liefert die Zielprodukte (Formel I) in ausgezeichneter Qualität und Ausbeute.

Selbstverständlich können die einzelnen Stufen des erfindungsgemäßen Verfahrens auch isoliert oder kombiniert, die Oxidation und Reduktion auch in Gegenwart gängiger Lösungs- oder Verdünnungsmittel, sofern sie unter Reaktionsbedingungen ausreichend stabil sind, durchgeführt werden. Der Vorteil besonders einfacher Verfahrensdurchführung im rein wäßrigen System und damit in technisch üblichen Apparaten geht dadurch aber eilweise verloren (Lösungsmittel-Filtration und/oder -Regeneration in speziellen Apparaturen), es treten durch Lösungsmittel-Verlust zusätzliche Kosten auf, ohne daß Ausbeute und/oder Qualität positiv beeinflußt werden. Eine Oxidation und/oder Reduktion in Gegenwart von Lösungs- oder Verdünnungmitteln ist daher nicht bevorzugt.

In der Europäischen Patentanmeldung 0 092 909 wird ein Verfahren zur Herstellung von Aminophenyl-ß-hydroxyethylsulfonen durch Umsetzung eines nicht weiter substituierten Nitrochlorbenzols mit Mercaptoethanol in Gegenwart von Alkalihydroxid und mindestens einem N-alkylsubstituierten Amid oder Sulfoxid als dipolar-aprotischem Lösungsmittel, Oxidation des gebildeten Sulfids und Reduktion der Nitrogruppe beschrieben. Das aprotische Lösungsmittel kann zwar einen geringen Wassergehalt aufweisen, übersteigt dieser aber einem bestimmten Prozentgehalt, so verlangsamt sich die Reaktion und unerwünschte Nebenreaktionen werden begünstigt. Demgegenüber zeichnet sich das erfindungsgemäße Verfahren dadurch aus, daß durch das Arbeiten in rein wäßrigem Medium der Aufwand für die Entfernung der organischen Lösungsmittel bzw. bei einem Belassen dieser Lösungsmittel die damit verbundene Umweltbelastung entfällt.

Die nachfolgenden Beispiele sollen das Verfahren näher erläutern, ohne es darauf einzuschränken.

## Beispiel 1

Eine Mischung aus 347 Teilen 5-Chlor-2-nitrophenol, 300 Teilen Thioglykol und 200 Teilen Wasser wird auf 70-75 °C geheizt und bei dieser Temperatur unter Rühren und Sauerstoffausschluß innerhalb von 3 Stunden in Anteilen mit 350 Teilen festem Kaliumcarbonat versetzt. Man rührt 6-8 Stunden nach, bis durch dünnschichtchromatografische Kontrolle kein Ausgangsprodukt mehr nachweisbar ist, kühlt auf Raumtemperatur ab und stellt durch Zutropfen von ca. 425 Teilen 30 %iger Salzsäure auf pH 4, wobei das 2-Nitrophenol-5-oxethylsulfid in gelben Kristallen ausfällt. Man isoliert es bei 10-15 °C durch Absaugen auf einer Nutsche und erhält nach Neutralwaschen und Trocknen im Vakuum bei 50-60 °C 394 Teile 2-Nitrophenol-5-oxethylsulfid vom Schmelzpunkt 85-86 °C, was einer Ausbeute von 91,6 % d. Th., bezogen auf 5-Chlor-2-nitrophenol, entspricht.

Das Produkt ist chromatografisch rein und zeigt in der Elementaranalyse die seiner Konstitution entsprechenden Werte (berechnet : $NO_2$ : 21,4 % ; S : 14,9 % ; OH : 7,9 % ; Cl : 0,0 % ; gefunden : $NO_2$ : 21,4/21,5 % ; S : 15,0 % ; OH : 7,9/8,1 % ; Rest-Cl : 0,012 %).

Verwendet man an Stelle von Kaliumcarbonat entsprechende Mengen Natriumcarbonat, so muß die Eintrag- und Nachrührzeit um ca. 50 % verlängert werden, um ein vergleichbares Ergebnis zu erhalten.

## Beispiel 2

Eine Mischung aus 435 Teilen 5-Chlor-2-nitrohydrochinondimethylether, 200 Teilen Thioglykol und 200 Teilen Wasser wird bei 55-60 °C unter Rühren und Sauerstoffausschluß innerhalb von 3 Stunden gleichmäßig mit 175 Teilen Kaliumcarbonat versetzt und anschließend 12-15 Stunden bei 60 °C nachgerührt, bis eine dünnschichtchromatografische Kontrolle vollständige Umsetzung anzeigt. Man kühlt auf Raumtemperatur und saugt den gelben Niederschlag auf einer Nutsche ab. Nach Neutralwäsche und Trocknen bei 60-80 °C im Vakuum erhält man 480 Teile 2-Nitro-hydrochinondimethylether-5-oxethylsulfid vom Schmelzpunkt 112-113 °C, was einer Ausbeute von 92,6 % d. Theorie, bezogen auf 5-Chlor-2-nitrohydrochinon-dimethylether, entspricht. Das Produkt ist chromatografisch rein und zeigt in der Elementaranalyse die seiner Konstitution entsprechenden Werte (berechnet : $NO_2$ : 17,8 % ; S : 12,4 % ; $OCH_3$ : 23,9 % ; Cl : 0,0 % ; gefunden : $NO_2$ : 18,0/17,8 % ; S : 12,4 % ; $OCH_3$ : 23,6/23,8 % ; Rest-Cl : 0,005 %).

Beispiel 3

Ersetzt man in Beispiel 2 den 5-Chlor-2-nitrohydrochinondimethylether durch eine aliquote Menge an 5-Chlor-2-nitrohydrochinon-diethylether und arbeitet sonst in der angegebenen Weise, so erhält man 260 Teile 2-Nitro-hydrochinon-diethylether-5-oxethylsulfid von Schmelzpunkt 108-110 °C in etwas geringerer Qualität (Rest-Cl-Gehalt : 0,3-0,4 %), der sich als solcher ohne Einschränkung zur Oxidation einsetzen läßt. Die Ausbeute, bezogen auf eingesetzten 5-Chlor-2-nitrohydrochinon-diethylether, beträgt 90,8 % d. Theorie.

Beispiel 4

Zu einer Mischung aus 403 Teilen 2-Chlor-5-nitro-p-kresolmethylether, 185 Teilen Thioglykol und 220 Teilen Wasser wird bei 75-80 °C unter Rühren und Sauerstoffausschluß im Verlaufe von 5 Stunden eine bei 80 °C gesättigte wäßrige Lösung von 175 Teilen Kaliumcarbonat gleichmäßig zugetropft. Man rührt bis zur Beendigung der Reaktion (4-6 Stunden, Kontrolle durch Dünnschichtchromatografie) bei 80-85 °C nach, kühlt, auf Raumtemperatur ab und isoliert den ausgefallenen gelben Niederschlag auf einer Nutsche.

Nach Neutralwaschen und Trocknen im Vakuum bei 60 °C erhält man 432 Teile 3-Nitro-4-methoxytoluol-6-oxethylsulfid vom Schmelzpunkt 98-99 °C, was einer Ausbeute von 88,8 % der theorie, bezogen auf 2-Chlor-5-nitro-p-kresolmethylether, entspricht. Das Produkt ist chromatografisch nahezu einheitlich und als solches für die nachfolgende Oxidation ohne Einschränkung einsetzbar (Rest-Cl-Gehalt : 0,2-0,25 %).

Beispiel 5

Eine Mischung aus 215 Teilen 2-Nitrophenol-5-oxethylsulfid, 4 Teilen Natriumwolframat-dihydrat und 1 000 Teilen Wasser wird bei 60 °C mit 20 %iger Essigsäure auf pH 5-5,5 eingestellt und dann im Verlaufe von 30 Minuten mit 117 Teilen 30 %igem Wasserstoffperoxid versetzt, wobei gleichzeitig die Innentemperatur auf 80 °C gesteigert wird. Anschließend läßt man bei 80-90 °C innerhalb einer weiteren Stunde nochmals gleichmäßig 152 Teile 30 %iges Wasserstoffperoxid zutropfen, wobei eine klare, nahezu farblose Lösung entsteht. Man rührt 3 Stunden bei 90-95 °C nach (Kontrolle auf vollständige Umsetzung durch Dünnschichtchromatografie) und kühlt dann unter Rühren auf 10-15 °C ab. Die sich dabei abscheidenden Kristalle werden abgesaugt, mit Wasser 2 mal abgedeckt und im Vakuum bei 60-80 °C getrocknet.

Man erhält 234 Teile 5-Oxethylsulfonyl-2-nitrophenol vom Schmelzpunkt 140-142 °C, was einer Ausbeute von 94,7 % d. Theorie, bezogen auf Einsatzprodukt, entspricht.

Die Verbindung ist chromatografisch rein und entspricht in der Elementaranalyse der angegebenen Konstitution (berechnet : $NO_2$ : 18,6 % ; S : 13,0 % ; gefunden : $NO_2$ : 18,6/18,6 % ; S : 12,7 %).

Beispiel 6

Zu einer gerührten Vorlage aus 229 Teilen 2-Nitroanisol-5-oxethylsulfid, Fp. 95-96 °C (hergestellt analog Beispielen 1-4 unter Einsatz von 2-Nitro-5-chloranisol), 4,5 Teilen Kaliumwolframat und 1 200 Teilen Wasser, die mit 2 n Salzsäure auf pH 4,5-5 eingestellt ist, werden in 1 Stunde bei 70-75 °C 100 Teile 35 %iges Wasserstoffperoxid getropft. Man heizt in 30 Minuten auf 90-95 °C und tropft weitere 110 Teile 35 %iges Wasserstoffperoxid in 90 Minuten zu, rührt 3-4 Stunden bei dieser Temperatur nach (Kontrolle auf vollständige Umsetzung durch HPLC), kühlt dann auf 20-25 °C ab und isoliert den ausgefallenen, gelblich-weißen Niederschlag durch Filtration auf einer Nutsche. Man deckt zweimal mit Wasser ab, saugt trocken und trocknet im Vakuum bei 60-70 °C. Es resultieren 250 Teile 5-Oxethylsulfonyl-2-nitroanisol vom Schmelzpunkt 106-108 °C, entsprechend einer Ausbeute von 95,8 % d. Th., bezogen auf Einsatzprodukt.

Die Verbindung ist chromatografisch rein und zeigt in der Elementaranalyse seiner Konstitution entsprechende Werte (berechnet : $NO_2$ : 17,6 % ; S : 12,3 % ; gefunden : $NO_2$ : 17,5 % ; S : 12,0/12,3 %).

Beispiel 7

Eine mit 20 %iger Phosphorsäure auf pH 5-5,5 gestellte Mischung aus 518 Teilen 2-Nitrohydrochinondimethylether-5-oxethylsulfid, 2 500 Teilen Wasser und 6 Teilen Wolframtrioxid wird bei 55-60 °C innerhalb von 45 Minuten unter Rühren gleichmäßig mit 90 Teilen 80 %igem Wasserstoffperoxid versetzt, wobei die Temperatur bis maximal 75 °C ansteigen darf. Anschließend wird 1 Stunde bei 75 °C nachgerührt und bei dieser Temperatur innerhalb weiterer 90 Minuten nochmals 100 Teile 80 %iges Wasserstoffperoxid tropfenweise zugegeben. Man steigert die Innentemperatur auf 90-95 °C und führt die Reaktion durch 3-4 stündiges Nachrühren zu Ende (Kontrolle durch HPLC). Anschließend läßt man abkühlen, saugt den ausgefallenen Niederschlag bei 15-20 °C auf einer Nutsche ab, wäscht zweimal mit Eiswasser und trocknet im Umluftschrank bei 60-70 °C. Man erhält 561 Teile nahezu farblosen 5-Oxethylsulfonyl-2-nitrohydrochinon-dimethylether vom Schmelzpunkt 129-131 °C, was einer Ausbeute von 96,4 % d. Th. bezogen auf Einsatzprodukt, entspricht.

Die Verbindung ist chromatografisch rein und liefert in der Elementaranalyse konstitutionsgemäße Werte (berechnet : $NO_2$ : 15,8 % ; S : 11,0 % ; gefunden : $NO_2$ : 16,0/15,8 % ; S : 11,0 %).

Wird die Oxidation in Abwesenheit des Wolfram-Katalysators, aber sonst in der angegebenen Weise durchgeführt, so ist eine viermal längere Reaktionszeit und ein um 10-15 % höherer Wasserstoffperoxid-Bedarf erforderlich. Qualität und Ausbeute werden dadurch nicht verändert.

## Beispiel 8

Eine Mischung aus 173,5 Teilen 5-Chlor-2-nitrophenol, 110 Teilen Thioglykol und 100 Teilen Wasser wird bei 80-85 °C unter Rühren und Sauerstoffausschluß in 5 Stunden portionsweise mit 152 Teilen Kaliumcarbonat versetzt. Man rührt unter langsamer Temperatursteigerung auf 90 °C ca. 5-6 Stunden nach, bis kein Ausgangsprodukt mehr nachweisbar ist (Kontrolle durch Dünnschichtchromatografie), setzt 900 Teile Wasser und 4 Teile Natriumwolframatdihydrat zu und stellt die resultierende Lösung mit 85 %iger Phosphorsäure auf pH 5-5,5 ein.

Nachfolgend tropft man in 60 Minuten bei 60 °C beginnend 210 Teile 30 %iges Wasserstoffperoxid zu, wobei die Temperatur auf 75-80 °C ansteigen soll. Anschließend läßt man bei 80-90 °C innerhalb einer weiteren Stunde nochmals gleichmäßig 160 Teile 30 %iges Wasserstoffperoxid zutropfen, rührt 3-4 Stunden bei 90-95 °C nach (Kontrolle auf vollständige Umsetzung durch HPLC) und kühlt dann unter Rühren auf 10-15 °C ab. Das dabei ausfallende Produkt wird abgesaugt, mit Wasser neutral und salzfrei gewaschen und im Vakuum bei 60-80 °C getrocknet.

Man erhält 225 Teile 5-Oxethylsulfonyl-2-nitrophenol vom Schmelzpunkt 140-141 °C, was einer Ausbeute von 91,1 % d. Theorie, bezogen auf 5-Chlor-2-nitrophenol, entspricht.

## Beispiel 9

Eine Suspension, bestehend aus 289,9 Teilen 5-Brom-2-nitrohydrochinon-diethylether, 100 Teilen Thioglykol und 300 Teilen Wasser wird bei 65-70 °C unter Rühren und Sauerstoffausschluß in 4 Stunden portionsweise mit 75 Teilen Kaliumcarbonat versetzt. Man rührt 8-10 Stunden bei 70-75 °C nach (Kontrolle auf vollständige Umsetzung durch Dünnschichtchromatografie), verdünnt den Ansatz mit 800 Teilen Wasser, setzt 5 Teile Kaliumwolframat zu und stumpft mit 50 %iger Essigsäure auf pH 5-5,5 ab.

Bei 50-55 °C beginnend werden nun in 60 Minuten 130 Teile 35 %iges Wasserstoffperoxid zugetropft, wobei die Temperatur auf maximal 75 °C ansteigen darf, und eine Stunde bei 75 °C nachgerührt. Daraufhin werden in 90 Minuten bei 75 °C nochmals 120 Teile 35 %iges Wasserstoffperoxid zugetropft, nachfolgend die Temperatur auf 90-95 °C gesteigert und bis zur vollständigen Oxidation (Kontrolle durch HPLC, Dauer ca. 4-5 Stunden) bei dieser Temperatur nachgerührt. Man läßt abkühlen, saugt den ausgefallenen Niederschlag bei 15-20 °C auf einer Nutsche ab, wäscht zweimal mit 200 Teilen Eiswasser und trocknet im Vakuum bei 50-65 °C.

Man erhält 288 Teile 5-Oxethylsulfonyl-2-nitrohydrochinondiethylether vom Schmelzpunkt 128-130 °C, was einer Ausbeute von 90,3 % d. Theorie, bezogen auf 5-Brom-2-nitrohydrochinon-diethylether, entspricht.

## Beispiel 10

In eine Mischung aus 187,5 Teilen 5-Chlor-2-nitroanisol, 120 Teilen Thioglykol und 120 Teilen Wasser werden bei 70-75 °C innerhalb 3 Stunden unter Rühren und Sauerstoffausschluß 70 Teile Kaliumcarbonat gleichmäßig eingetragen. Man rührt 6-8 Stunden bei 75-80 °C nach, bis kein Ausgangsprodukt mehr nachweisbar ist (Kontrolle durch Dünnschichtchromatografie), setzt dann 800 Teile 80 °C warmes Wasser und 3 Teile Wolframtrioxid zu und stellt durch Zutropfen von Essigsäure einen pH-Wert von 4-4,5 ein. Nun werden bei 80 °C innerhalb 30 Minuten 175 Teile 30 %iges Wasserstoffperoxid zugetropft, 1 Stunde unter langsamer Temperatur-Erhöhung auf 90-95 °C nachgerührt und dann in wiederum 30 Minuten weitere 150 Teile 30 %iges Wasserstoffperoxid zugetropft. Die Oxidation wird durch Nachrühren bei 90-95 °C zu Ende geführt (Kontrolle durch HPLC), das Produkt durch Abkühlen auf 20-25 °C abgeschieden, durch Filtration isoliert und nach Waschen mit Wasser im Vakuum bei 50-60 °C getrocknet.

Man erhält 230 Teile 5-Oxethylsulfonyl-2-nitroanisol vom Schmelzpunkt 105-107 °C, was einer Ausbeute von 92,5 % d. Th., bezogen auf 5-Chlor-2-nitroanisol, entspricht.

## Beispiel 11

Im Hydrierautoklav werden 760 Teile Wasser vorgelegt, nacheinander 98,8 Teile 5-Oxethylsulfonyl-2-nitrophenol und 2,5 Teile Edelmetall-Katalysator (5 % Pd/Kohle) eingetragen und durch Zugabe von Phosphatpuffer ein pH-Wert von 5,5-6 eingestellt.

Man verschließt den Autoklav, spült mit Stickstoff und anschließend Wasserstoff und hydriert dann

bei 15 bar Wasserstoffdruck/50-70 °C. Wenn kein Wasserstoff mehr aufgenommen wird, was nach ca. 60 Minuten der Fall ist, wird entspannt, bei 80 °C vom Katalysator geklärt und unter Sauerstoffausschluß auf 0-5 °C gekühlt. Man filtriert, wäscht mit 50-100 Teilen Eiswasser und trocknet im Vakuum bei 60-70 °C. Es werden 75 Teile 5-Oxethylsulfonyl-2-aminophenol vom Schmelzpunkt 115-117 °C erhalten, was einer Ausbeute von 86,4 % d. Th., bezogen auf eingesetzte Nitroverbindung, entspricht.

Aus der Mutterlauge lassen sich durch Zugabe von 10 % Kochsalz weitere 6 Teile Produkt ausfällen und durch Filtration isolieren, so daß eine Gesamtausbeute an 5-Oxethylsulfonyl-2-aminophenol von 81 Teilen, entsprechend 93,3 % d. Th. resultiert.

Eine vergleichbare Ausbeute wird auch erhalten, wenn beim nächsten Ansatz an Stelle des Wassers 760 Teile der Mutterlauge dieses Ansatzes vorgelegt und sonst in der angegebenen Weise gearbeitet wird.

Das isolierte 5-Oxethylsulfonyl-2-aminophenol ist chromatografisch rein. Sein Diazotierwert liegt bei 99,2 %. Das Produkt ist ohne Einschränkungen zur Herstellung von Reaktivfarbstoffen einsetzbar.

## Beispiel 12

Eine Mischung aus 200 Teilen Eisenspänen, 1 800 Teilen Wasser und 35 Teilen 30 %iger Salzsäure wird unter Rühren auf 95-100 °C geheizt. Dann werden in 2-3 Stunden gleichmäßig 247 Teile 5-Oxethylsulfonyl-2-nitrophenol eingetragen und 1 Stunde bei 95-100 °C nachgerührt. Die Reduktion ist beendet, wenn der Auslauf einer Tüpfelprobe auf Filterpapier farblos ist. Man stellt mit ca. 320 Teilen 33 %iger Natronlauge deutlich phenolphthalein-alkalisch, saugt den Eisenschlamm auf einer beheizten Nutsche ab und wäscht den Filterrückstand mit 500 Teilen 100 °C heißer 2 %igen Natronlauge. Man kühlt das vereinigte Filtrat unter Sauerstoffausschluß auf 40 °C, stellt mit 30 %iger Salzsäure einen pH-Wert von 5,0 ein und kühlt langsam auf 0 °C ab. Nach Absaugen und Trocknen bei 60-70 °C im Vakuum erhält man 191 Teile 5-Oxethylsulfonyl-2-aminophenol vom Schmelzpunkt 115-116 °C, was einer Ausbeute von 88,0 % d. Th., bezogen auf eingesetzte Nitroverbindung entspricht.

Aus der Mutterlauge läßt sich durch Aussalzen weiteres produkt vergleichbarer Qualität isolieren. Vorteilhaft kann aber auch die nach Klären vom Eisenschlamm erhaltene Lösung, nach Gehaltsbestimmung (Diazotierwert) ca. 98-99 % d. Th. an 5-Oxethylsulfonyl-2-aminophenol enthaltend, direkt und verlustfrei diazotiert und zum Farbstoff weiterverarbeitet werden. Aus diese Weise lassen sich die wegen der guten Wasserlöslichkeit von 5-Oxethylsulfonyl-2-aminophenol bei der Isolierung unvermeidlichen Produktverluste auf ein Minimum reduzieren.

## Beispiele 13-17

Analog den Beispielen 11 und/oder 12 werden auch die erfindungsgemäß zugänglichen 5-Oxethylsulfonyl-2-nitrophenolether (vgl. Beispiele 6, 7, 9 und 10) reduziert und die in nachfolgender Tabelle mit Schmelzpunkt, Reingehalt (Diazotierwert) und isolierter Ausbeute aufgeführten Produkte erhalten (in den Reduktionslösungen werden nahezu quantitative Ausbeuten mittels Diazotierwert und/oder potentiometrischer Titration nachgewiesen) :

Tabelle :

| Beispiel | Zielverbindung der Formel I | | Ausbeute | Reingehalt | Schmelzpunkt |
|---|---|---|---|---|---|
| | R | $R_1$ | | | |
| 13 | $CH_3$ | $OCH_3$ | 91,2 % | 99,4 % | 141 - 144°C |
| 14 | $CH_3$ | $CH_3$ | 91,6 % | 99,0 % | 84 - 87°C |
| 15 | $CH_3$ | H | 89,2 % | 99,0 % | 180 - 181°C (Hydrochlorid) |
| 16 | $C_2H_5$ | $OC_2H_5$ | 88,8 % | 98,8 % | 137 - 139°C |
| 17 | $C_2H_5$ | $CH_3$ | 89,5 % | 99,3 % | 81 - 82°C |

**Patentansprüche**

1. Verfahren zur Herstellung von 5-Oxethylsulfonyl-2-aminophenol(-ethern) der allgemeinen Formel (I)

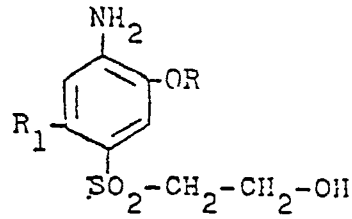

(I)

in welcher R ein Wasserstoffatom oder eine Alkylgruppe von 1-4 Kohlenstoffatomen, und $R_1$ ein Wasserstoffatom oder eine Alkyl- oder Alkoxygruppe von jeweils 1-4 Kohlenstoffatomen bedeuten, dadurch gekennzeichnet, daß man 5-Chlor-(oder Brom)-2-nitrophenole oder deren Alkylether der allgemeinen Formel (II)

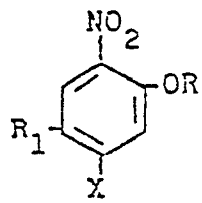

(II)

in welcher R und $R_1$ die vorstehend genannten Bedeutungen haben und X ein Chlor- oder Bromatom bedeutet, mit Thioglykol zu 2-Nitrophenol(-ether)-5-oxethylsulfiden der Formel (III)

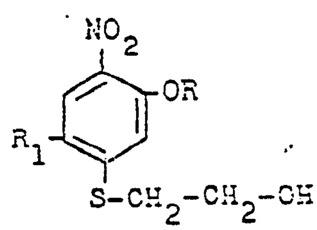

(III)

in welcher R und $R_1$ die vorstehend genannten Bedeutungen haben, in rein wäßrigem Medium kondensiert, diese, vorzugsweise ohne Zwischenisolierung, zu 5-Oxethylsulfonyl-2-nitrophenol(-ethern) der Formel (IV)

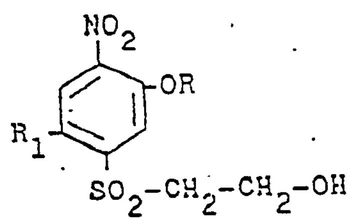

(IV)

oxidiert und letztere zu den Verbindungen der genannten Formel (I) reduziert.

2. Verfahren nach Anspruch 1, dadurch gekennzeichnet, daß man eine p-Nitrochlorbenzolverbindung der Formel (II) zusammen mit der mindestens stöchiometrischen Menge Thioglykol und soviel Wasser, daß ausreichende Rührbakeit gewährleistet ist, auf Temperaturen von 50-90 °C erhitzt, dann eine säurebindende Alkali- oder Erdalkalimetallverbindung in der mindestens stöchiometrischen Menge in Anteilen zugibt und nach Beendigung der Umsetzung mittels Säure einen pH-Wert von 4-6 einstellt.

3. Verfahren nach Anspruch 1, dadurch gekennzeichnet, daß man die in suspendierter Form angefallene Verbindung der Formel (III), vorzugsweise ohne Zwischenisolierung, nach Zusatz katalytischer Mengen Wolframtrioxid oder Alkalimetallwolframat und nach ggfs. erfolgtem Verdünnen mit Wasser mit einer mindestens stöchiometrische Menge Wasserstoffperoxid in Anteilen versetzt und nach

beendeter Oxidation die erhaltene Verbindung der Formel (IV) abtrennt.

## Claims

1. A process for the preparation of a 5-hydroxyethylsulfonyl-2-aminophenol(ether) of the formula (I)

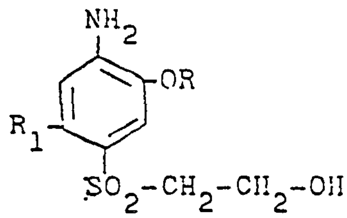

(I)

in which R denotes a hydrogen atom or an alkyl group with 1-4 carbon atoms and $R_1$ denotes a hydrogen atom or an alkyl or alkoxy group with in each case 1-4 carbon atoms, which comprises condensing a 5-chloro (or bromo)-2-nitrophenol or alkyl ether thereof of the formula (II)

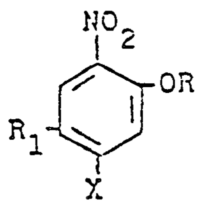

(II)

in which R and $R_1$ have the abovementioned meanings and X denotes a chlorine or bromine atom, with thioglycol to give a 2-nitrophenol(ether) 5-hydroxyethyl-sulfide of the formula (III)

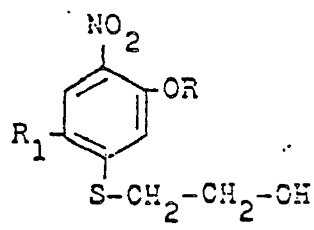

(III)

in which R and $R_1$ have the abovementioned meanings, oxidizing this product, preferably without intermediate isolation, to give a 5-hydroxyethylsulfonyl-2-nitrophenol(ether) of the formula (IV)

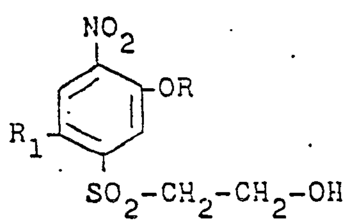

(IV)

and reducing the latter to give a compound of the above formula (I).

2. The process as claimed in claim 1, wherein a p-nitrochlorobenzene compound of the formula (II) is heated to a temperature of 50-90 °C with at least the stoichiometric amount of thioglycol and sufficient water to ensure adequate stirrability, at least the stoichiometric amount of an acid-binding alkali metal compound or alkaline earth metal compound is then added in portions and, when the reaction has ended, the pH value is brought to 4-6 by means of acid.

3. The process as claimed in claim 1, wherein at least a stoichiometric amount of hydrogen peroxide is added in portions to the compound of the formula (III), obtained in suspended form, preferably without intermediate isolation, after addition of catalytic amounts of tungsten trioxide or an alkali metal tungstate and, if appropriate, after dilution with water, and, when the oxidation has ended, the resulting compound of the formula (IV) is separated off.

**Revendications**

1. Procédé pour la préparation d'hydroxyéthylsulfonyl-5 amino-2 phénols (et de leurs éthers) de formule générale (I)

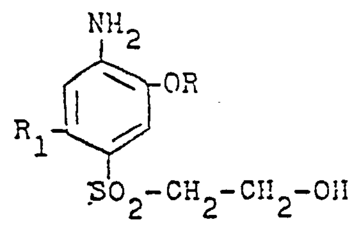

(I)

dans laquelle R est un atome d'hydrogène ou un groupe alkyle ayant de 1 à 4 atomes de carbone, et $R_1$ est un atome d'hydrogène ou un groupe alkyle ou alcoxy ayant chacun de 1 à 4 atomes de carbone, caractérisé en ce qu'on condense des chloro (ou bromo) -5 nitro-2 phénols ou leurs éthers alkyliques, de formule générale (II)

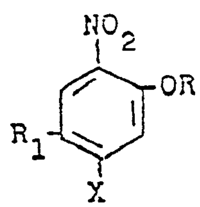

(II)

dans laquelle R et $R_1$ ont les significations données ci-dessus et X est un atome de chlore ou de brome, avec du thioglycol pour obtenir des hydroxyéthylthio-5 nitro-2 phénols (ou leurs éthers de formule (III)

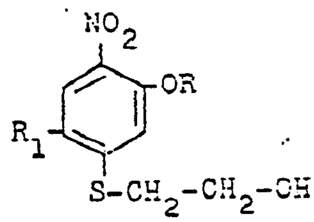

(III)

dans laquelle R et $R_1$ ont les significations données ci-dessus, en milieu purement aqueux, puis qu'on oxyde ces derniers, de préférence sans isolement intermédiaire, pour obtenir des hydroxyéthylsulfonyl-5 nitro-2 phénols (et leurs éthers) de formule (IV)

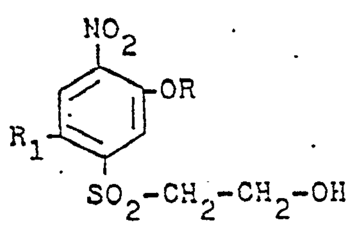

(IV)

et qu'on réduit ces derniers en les composés ayant la formule (I) mentionnée ci-dessus.

2. Procédé selon la revendication 1, caractérisé en ce qu'on chauffe à des températures de 50-90 °C un composé de p-nitrochlorobenzène de formule (II), en même temps qu'une quantité au moins stœchiométrique de thioglycol et d'une quantité d'eau suffisante pour assurer une agitabilité suffisante, puis qu'on ajoute un composé d'un métal alcalin ou d'un métal alcalino-terreux fixant les acides, par portions, en une quantité au moins stœchiométrique, le pH étant ajusté à 4-6 à l'aide d'un acide quand la réaction est terminée.

3. Procédé selon la revendication 1, caractérisé en ce qu'on ajoute par portions une quantité au moins stœchiométrique de peroxyde d'hydrogène au composé de formule (III) obtenu en suspension, de préférence sans isolement intermédiaire, après addition de quantités catalytiques de trioxyde de tungstène ou de tungstate d'un métal alcalin, et éventuellement après une dilution à l'eau, puis, après achèvement de l'oxydation, qu'on sépare le composé de formule (IV) tel qu'obtenu.